# EUROPEAN PATENT SPECIFICATION

(11) **EP 1 673 329 B1**
(45) Date of publication and mention of the grant of the patent: **12.09.2007**
(21) Application number: 03772649.4
(22) Date of filing: 13.10.2003
(51) Int. Cl.: C07C 29/40, C07C 33/46, C07C 17/16, C07C 22/08, C07F 3/02

(54) **PROCESS FOR THE PREPARATION 3,5-BIS(TRIFLUOROMETHYL)BENZYLALCOHOL**
VERFAHREN ZUR HERSTELLUNG VON 3,5-BIS(TRIFLUORMETHYL)BENZYLALKOHOL
PREPARATION D'ALCOOL 3,5-BIS(TRIFLUOROMETHYL) BENZYLIQUE

(43) Date of publication of application: 28.06.2006
(73) Proprietor: MITENI S.p.A., 20138 Milan (IT)
(72) Inventor: NARDELLO, Alessandro, c/o Miteni S.p.A., I-36070 Trissino (VI) (IT); PRETTO, Marisa, c/o Miteni S.p.A., I-36070 Trissino (VI) (IT); FACCIN, Andrea, c/o Miteni S.p.A., I-36070 Trissino (VI) (IT)
(74) Representative: Trupiano, Federica
(86) International application number: PCT/IT2003/000616
(87) International publication number: WO 2005/035472

(56) References cited:
- EP-A- 0 491 326
- WO-A-02/088058
- US-A- 3 625 970

## Description

### BACKGROUND OF THE INVENTION

This invention concerns a new process for preparing 1,3-bis(trifluoromethyl)benzene derivatives. In particular, the invention concerns a process for preparing 3,5-bis(trifluoromethyl)benzyl alcohol by formylation of 3,5-bis(trifluoromethyl)phenyl-magnesium halide with paraformaldehyde.

3,5-bis(trifluoromethyl)benzylalcohol is also a very useful intermediate in the preparation of 3,5-bis(trifluoromethyl)halo-benzyls, for example 3,5-bis(trifluoromethyl)benzylbromide or benzylchloride.

The United States patent US 3,625,970 describes the preparation of 3,5-bis(trifluoromethyl)benzylalcohol by reaction of 3,5-bis(trifluoromethyl)-phenyl magnesium halides with gaseous formaldehyde produced by thermal decomposition of paraformaldehyde according to the description given in Organic Synthesis, Vol I, pages 188-190. In particular the latter article describes the formylation of halides of cyclohexyl-magnesium in diethyl ether, a solvent conventionally used for these formylation reactions, and it is specifically indicated that the use of paraformaldehyde leads to low yields, of 40-50%.

Though gaseous formaldehyde is a first choice reagent according to the known technique, it is nevertheless highly unadvisable due to its toxicity and the critical conditions of handling.

### BRIEF DESCRIPTION OF THE INVENTION

It has now been surprisingly found that the formylation reaction of organo-magnesium 1,3-bis(trifluoromethyl)benzene derivatives with solid paraformaldehyde may be carried out with ease and allows to obtain on this substratum yields in 3,5-bis(trifluoromethyl)benzylalcohol comparable to those obtained with gaseous formaldehyde.

### DETAILED DESCRIPTION OF THE INVENTION

So, according to one of its aspects, the invention concerns a process for preparing 3,5-bis(trifluoromethyl)benzyl alcohol which comprises reacting a 3,5-bis(trifluoromethyl)-phenylmagnesium halide with solid paraformaldehyde in a solvent.

According to the present invention, appropriate solvents are aliphatic ethers such as, for example, tetrahydrofuran (THF), alone or, preferably, in admixture with aromatic hydrocarbons. For example, mixtures of diethyl ether, THF, methyl-THF, isobutyl-ether, dimetoxyethane (DME), diethoxyethane, diglyme, butyl-diglyme, ethyl-diglyme, triglyme with toluene, o,m,p-xylenes, o,m,p-hexafluoroxylenes for example 1,3-bis(trifluoromethyl)benzene, and similar may be used.

According to a preferred aspect, a mixture of THF and an aromatic hydrocarbon is used, for example toluene or 1,3-bis(trifluoromethyl)benzene, advantageously a mixture which comprises from 20 to 60% p/p of THF.

According to the invention the tetrahydrofuran is substantially anhydrous.

The starting 3,5-bis(trifluoromethyl)-phenylmagnesium halide according to the invention may be obtained from the corresponding 3,5-bis(trifluoromethyl)-1-halobenzene by treatment with magnesium according to the conventional techniques well known to the skilled in the art.

For example the preparation of the 3,5-bis(trifluoromethyl)-phenylmagnesium halide may be carried out in any of the conditions known in the art for preparing organo-magnesium adducts (as described for example in Organic Synthesis, vol. 1, page 550; Chem. Ber. 1996, 129:233-235 and related references); the reaction is advantageously carried out in an anhydrous environment to avoid the hydrolysis of the organo-magnesium adduct, at a temperature between room temperature and the reflux temperature of the mixture of solvents. An excess of magnesium is generally used. It is not usually necessary to activate the reaction, since the addition for example of 3,5-bis(trifluoromethyl)-1-bromobenzene to the reaction mixture provokes self-starting; However, if necessary or desired, it is possible to add a usual activator, such as bromine, iodine, 1,2-dibromoethane or Vitride®, in order to accelerate the starting of the reaction. Generally after 2-5 hours all the reagent is used up; the progress of the reaction may be controlled by means of the usual gaschromatographic analytical controls or with thin layer liquid chromatography.

For example, the preparation between the starting 3,5-bis(trifluoromethyl)-phenylmagnesium halide from 3,5-bis(trifluoromethyl)-1-halobenzene may be carried out in one of the solvents indicated above for the preparation of 3,5-bis(trifluoromethyl)benzylalcohol. In this way it is possible to proceed with the synthesis of the 3,5-bis(trifluoromethyl)benzylalcohol according to the present invention without isolating the 3,5-bis(trifluoromethyl)-phenylmagnesium halide.

3,5-bis(trifluoromethyl)-1-bromobenzene and the starting 3,5-bis(trifluoromethyl)-1-chlorobenzene are known products, available on the market.

In practice, after having prepared the organo-magnesium derivative according to the known techniques as indicated above, powdered paraformaldehyde is added to the reaction mass, advantageously in portions, and it is left to react for a period of between 1 and 5 hours, preferably at a temperature between 30 and 90°C.

According to the process of the invention, the amount of solid paraformaldehyde necessary to obtain the best yields is generally equimolar or slightly in excess with respect to the starting organo-magnesium derivative, for example an excess of not more than 5%. In fact it was noted with surprise that a great excess of this reagent, contrary to what is normally remarked in organic chemical reactions, does not lead to an improvement of yields, but rather leads to a decrease of the same and to a more difficult processing of the final reaction mixture.

When the reaction is ended (which can be checked by the techniques known to the skilled in the art, for example by gas-chromatography), the adduct formed is hydrolysed with an aqueous solution of a mineral acid, such as sulphuric acid or hydrochloric acid, and the desired product, 3,5-bis(trifluoromethyl)benzylalcohol, is obtained, which may be isolated and purified with the usual techniques, for example by crystallising or distilling, or used as it is for further chemical transformations.

In fact 3,5-bis(trifluoromethyl)benzylalcohol, isolated and purified or as a crude reaction product, can in turn be used as the starting product for the preparation of halogenated derivatives such as 3,5-bis(trifluoromethyl)benzyl halides, 3,5-bis(trifluoromethyl)benzyl bromide or 3,5-bis(trifluoromethyl)benzyl chloride.

To do this it is sufficient to submit the 3,5-bis(trifluoromethyl)benzylalcohol to a halogenation reaction, for example with aqueous hydrochloric acid or hydrobromic acid, optionally in the presence of sulphuric acid.

Thus, according to another of its aspects, the invention concerns a process for preparing 3,5-bis(trifluoromethyl)benzyl halides, which comprises:
(a) forming a 3,5-bis(trifluoromethyl)-phenyl magnesium halide from a 3,5-bis(trifluoromethyl)-halo-benzene in a solvent selected from the aliphatic ethers and a mixture of aliphatic ethers and aromatic hydrocarbons.
(b) adding solid paraformaldehyde to the reaction mixture thus obtained;
(c) submitting the 3,5-bis(trifluoromethyl)benzylalcohol thus obtained to a halogenation reaction with HX where X is a halide, optionally in the presence of sulphuric acid;
(d) isolating the 3,5-bis(trifluoromethyl)benzyl halide thus obtained.

The starting halogenated derivative of step (a) and the HX acid of step (c) need not necessarily contain the same halogen.

According to a preferred embodiment, in step (a) 3,5-bis(trifluoromethyl)-phenylmagnesium bromide is used or 3,5-bis(trifluoromethyl)-phenylmagnesium chloride, the bromine derivative being particularly preferred.

According to another preferred embodiment, in step (c) X is selected from bromide, chloride and iodide, bromide being a preferred halide.

The reaction in step (c) may also be carried out according to any appropriate technique for the halogenation of benzylalcohols, for example with phosphorous tribromide (PBr₃) or NaBr and sulphuric acid , by heating.

Some preferred embodiments of the invention are given in the following experimental part.

The following example is given purely as illustration and is not limiting in any way.

### EXPERIMENTAL PART

### EXAMPLE 1

### (i) Preparation of 3,5-bis(trifluoromethyl)-phenyl magnesium bromide

In a 4-neck flask with capacity 5 L equipped with mechanical stirrer, thermometer, bubble condenser and 250 ml filling filter , at room temperature 5.9 g of Mg (1.8881 moles); 310.0 g of anhydrous THF; 50.0 g of 3,5-bis(trifluoromethyl)-1-bromo-benzene (0.1706 moles) are loaded. Stirring vigorously, wait for the starting of the reaction (10 minutes approx.). A temperature increase is noted and a variation in the colour of the reaction mass from colourless to dark brown. A temperature increase up to about 60°C is noted. The exothermic reaction is allowed to abate and, as soon as a fall in the internal temperature is noted, 826 g of anhydrous 1,3-bis(trifluoromethyl)benzene are loaded in the flask. The remaining: 451.0 g of 3,5-bis(trifluoromethyl)-1-bromo-benzene (1.539 moles) are metered, regulating the pouring rate so as to maintain the temperature at 45°C (pouring duration 4.5 h). Once pouring is finished, 45°C are maintained for 1-3 h.

### (ii)-Preparation of 3,5-bis(trifluoromethyl)-benzylalcohol

53.8 g of solid paraformaldehyde (1,7933 moles) are added to the reaction mixture obtained in the previous step (i), dosing it in two portions of about 27g. The reaction is exothermic. It is left to react at 45°C for 6 hours and then the organo-magnesium adduct is hydrolysed with 1002.3 g of H₂SO₄ at 20%, cooling the system by means of an ice/water bath (Tₘₐₓ=37°C), after which it is kept stirring vigorously for another hour. the mixture is left to settle, the two phases are separated and the solvents are eliminated from the organic phase; it is then distilled in a vacuum (20 mbar) collecting 318.6 g of evaporated crude alcohol (tit. > 92%).

### EXAMPLE 2

### Preparation of 3,5-bis(trifluoromethyl)-benzyl bromide

In a 4-neck flask with capacity 1000 ml equipped with mechanical agitator, thermometer, bubble condenser and 100 ml loading funnel , 262.2 g of the product of Example 1 (ii) at 92.8% (0.988 moles), 550,2 g HBr 48% (3.2645 moles) are loaded; this is heated at 50°C so as to melt the alcohol, then one starts to dose 113 g of concentrated H₂SO₄ (1.153 moles). Pouring is accomplished in 30 minutes, noting an increase of the internal temperature. This is heated to 100-105°C and left to react for 8 hours. The reaction is completed by reflux heating for per 1.5 hours. the mixture is left to settle and the phases are separated; the solvents are removed from the organic phase and the product in the title is obtained with a yield of 99.1 %.

## Claims

1. Process for preparing 3,5-bis(trifluoromethyl)benzylalcohol which comprises reacting a 3,5-bis(trifluoromethyl)-phenylmagnesium halide with solid paraformaldehyde in a solvent.

2. Process according to claim 1, **characterised in that** said solvent is an aliphatic ether.

3. Process according to claim 2, **characterised in that** said aliphatic ether is tetrahydrofuran (THF).

4. Process according to claim 1, **characterised in that** said solvent is a mixture of aliphatic ethers and aromatic hydrocarbons .

5. Process according to claim 4, **characterised in that** the aliphatic ether is selected from diethyl ether, THF, methyl-THF, isobutyl-ether, dimetoxyethane (DME), diethoxyethane, diglyme, butyl-diglyme, ethyl-diglyme and triglyme.

6. Process according to claim 4, **characterised in that** the aromatic hydrocarbon is selected from toluene, o,m,p-xylenes, o,m,p- hexafluoroxylenes and 1,3-bis(trifluoromethyl)benzene.

7. Process according to claim 4, **characterised in that** the reaction solvent is a mixture of THF and an aromatic hydrocarbon.

8. Process according to claim 7, **characterised in that** the reaction solvent is a mixture of THF and an aromatic hydrocarbon selected from toluene and 1,3-bis(trifluoromethyl)benzene.

9. Process according to claim 7 or 8, **characterised in that** said mixture comprises from 20 to 60% p/p of THF.

10. Process according to any one of the previous claims, **characterised in that** the 3,5-bis(trifluoromethyl)-phenylmagnesium halide is selected from 3,5-bis(trifluoromethyl)-phenylmagnesium bromide and 3,5-bis(trifluoromethyl)-phenylmagnesium chloride.

11. Process according to any one of the previous claims, **characterised in that** the solid paraformaldehyde is used in an approximately equimolar amount or slightly in excess with respect to the 3,5-bis(trifluoromethyl)-phenyl magnesium halide.

12. Process according to claim 11 **characterised in that** the molar excess of paraformaldehyde is less than or equal to 5% with respect to the halide or 3,5-bis(trifluoromethyl)phenyl-magnesium.

13. Process according to any one of the previous claims, **characterised in that** the reaction temperature is between 30 and 90°C.

14. Process according to any one of the previous claims, **characterised in that** at the end of the reaction the adduct is hydrolysed with an aqueous solution of a mineral acid.

15. Process according to claim 14, **characterised in that** said mineral acid is selected from hydrochloric acid and sulphuric acid.

16. Process according to any one of the previous claims, **characterised in that** the 3,5-bis(trifluoromethyl)benzylalcohol is isolated by distillation or crystallisation.

17. Process according to any one of the previous claims, **characterised in that** the 3,5-bis(trifluoromethyl)benzylalcohol obtained is used as a reagent to obtain a 3,5-bis(trifluoromethyl)benzyl halide.

18. Process according to any one of the previous claims, **characterised in that** said 3,5-bis(trifluoromethyl)-phenyl-magnesium halide is obtained starting from the corresponding 3,5-bis(trifluoromethyl)-1-halobenzene by treatment with magnesium in a solvent selected from the solvents quoted in claims 2 to 9.

19. Process according to claims 17 and 18, **characterised in that**:
(a) a 3,5-bis(trifluoromethyl)-phenyl magnesium halide is formed from a 3,5-bis(trifluoromethyl)-halobenzene in a solvent selected from the aliphatic ethers and a mixture of aliphatic ethers and aromatic hydrocarbons;
(b) solid paraformaldehyde is added to the reaction mixture thus obtained;
(c) the 3,5-bis(trifluoromethyl)benzylalcohol thus obtained is submitted to a halogenation reaction with HX where X is a halide, optionally in the presence of sulphuric acid;
(d) the 3,5-bis(trifluoromethyl)benzyl halide thus obtained is isolated.

## Patentansprüche

1. Verfahren zur Herstellung von 3,5-Bis(trifluormethyl)benzylalkohol, das die Umsetzung eines 3,5-Bis(trifluormethyl)phenylmagnesiumhalogenids mit festem Paraformaldehyd in einem Lösungsmittel umfasst.

2. Verfahren nach Anspruch 1, **dadurch gekennzeichnet, dass** das Lösungsmittel ein aliphatischer Ether ist.

3. Verfahren nach Anspruch 2, **dadurch gekennzeichnet, dass** der aliphatische Ether Tetrahydrofuran (THF) ist.

4. Verfahren nach Anspruch 1, **dadurch gekennzeichnet, dass** das Lösungsmittel eine Mischung aus aliphatischen Ethern und aromatischen Kohlenwasserstoffen ist.

5. Verfahren nach Anspruch 4, **dadurch gekennzeichnet, dass** der aliphatische Ether ausgewählt ist aus Diethylether, THF, Methyl-THF, Isobutylether, Dimethoxyethan (DME), Diethoxyethan, Diglyme, Butyldiglyme, Ethyldiglyme und Triglyme.

6. Verfahren nach Anspruch 4, **dadurch gekennzeichnet, dass** der aromatische Kohlenwasserstoff ausgewählt ist aus Toluol, o,m,p-Xylolen, o,m,p-Hexafluorxylolen und 1,3-Bis(trifluormethyl)benzol.

7. Verfahren nach Anspruch 4, **dadurch gekennzeichnet, dass** das Reaktionslösungsmitel eine Mischung aus THF und einem aromatischen Kohlenwasserstoff ist.

8. Verfahren nach Anspruch 7, **dadurch gekennzeichnet, dass** das Reaktionslösungsmittel eine Mischung aus THF und einem aromatischen Kohlenwasserstoff ist, der ausgewählt ist aus Toluol und 1,3-Bis(trifluormethyl)benzol.

9. Verfahren nach Anspruch 7 oder 8, **dadurch gekennzeichnet, dass** die Mischung 20 bis 60% (p/p) THF umfasst.

10. Verfahren nach einem der vorhergehenden Ansprüche, **dadurch gekennzeichnet, dass** das 3,5-Bis(trifluormethyl)phenylmagnesiumhalogenid ausgewählt ist aus 3,5-Bis(trifluormethyl)phenylmagnesiumbromid und 3,5-Bis(trifluormethyl)phenylmagnesiumchlorid.

11. Verfahren nach einem der vorhergehenden Ansprüche, **dadurch gekennzeichnet, dass** das feste Paraformaldehyd in etwa äquimolarer Menge oder in einem leichten Überschuss gegenüber dem 3,5-Bis(trifluormethyl)phenyl-magnesiumhalogenid verwendet wird.

12. Verfahren nach Anspruch 11, **dadurch gekennzeichnet, dass** der molare Überschuss des Paraformaldehyds weniger als oder gleich 5% bezogen auf das Halogenid oder 3,5-Bis(trifluormethyl)phenylmagnesium ist.

13. Verfahren nach einem der vorhergehenden Ansprüche, **dadurch gekennzeichnet, dass** die Reaktionstemperatur zwischen 30 und 90°C liegt.

14. Verfahren nach einem der vorhergehenden Ansprüche, **dadurch gekennzeichnet, dass** das Addukt am Reaktionsende mit einer wässrigen Lösung einer Mineralsäure hydrolysiert wird.

15. Verfahren nach Anspruch 14, **dadurch gekennzeichnet, dass** die Mineralsäure ausgewählt ist aus Salzsäure und Schwefelsäure.

16. Verfahren nach einem der vorhergehenden Ansprüche, **dadurch gekennzeichnet, dass** der 3,5-Bis(trifluormethyl)benzylalkohol durch Destillation oder Kristallisation isoliert wird.

17. Verfahren nach einem der vorhergehenden Ansprüche, **dadurch gekennzeichnet, dass** der erhaltene 3,5-Bis(trifluormethyl)benzylalkohol als Reagens zur Gewinnung eines 3,5-Bis(trifluormethyl)benzylhalogenids verwendet wird.

18. Verfahren nach einem der vorhergehenden Ansprüche, **dadurch gekennzeichnet, dass** das 3,5-Bis(trifluormethyl)phenylmagnesiumhalogenid ausgehend vom entsprechenden 3,5-Bis(trifluormethyl)-1-halogenbenzol durch Behandlung mit Magnesium in einem Lösungsmittel erhalten wird, das ausgewählt ist aus den Lösungsmitteln, die in den Ansprüchen 2 bis 9 angeführt sind.

19. Verfahren nach den Ansprüchen 17 und 18, **dadurch gekennzeichnet, dass**:
a) ein 3,5-Bis(trifluormethyl)phenylmagnesiumhalogenid aus einem 3,5-Bis(trifluormethyl)halogenbenzol in einem Lösungsmittel gebildet wird, das ausgewählt ist aus aliphatischen Ethern und einer Mischung aus aliphatischen Ethern und aromatischen Kohlenwasserstoffen;
b) festes Paraformaldehyd zur so erhaltenen Reaktionsmischung gegeben wird;
c) der so erhaltene 3,5-Bis(trifluormethyl)benzylalkohol gegebenenfalls in Gegenwart von Schwefelsäure einer Halogenierungsreaktion mit HX unterzogen wird, worin X ein Halogenid ist;
d) das so erhaltene 3,5-Bis(trifluormethyl)benzylhalogenid isoliert wird.

## Revendications

1. Procédé de préparation de l'alcool 3,5-bis(trifluorométhyl)benzyle comprenant la réaction d'un halogénure du 3,5-bis(trifluorométhyl)phénylmagnésium avec du paraformaldéhyde solide dans un solvant.

2. Procédé selon la revendication 1, **caractérisé en ce que** ledit solvant est un éther aliphatique.

3. Procédé selon la revendication 2, **caractérisé en ce que** ledit éther aliphatique est le tétrahydrofurane (THF).

4. Procédé selon la revendication 1, **caractérisé en ce que** ledit solvant est un mélange d'éthers aliphatiques et d'hydrocarbures aromatiques.

5. Procédé selon la revendication 4, **caractérisé en ce que** l'éther aliphatique est choisi parmi le diéthyl éther, le THF, le méthyl-THF, l'isobutyl-éther, le diméthoxyéthane (DME), le diéthoxyéthane, le diglyme, le butyl-diglyme, l'éthyl-diglyme et le tryglyme.

6. Procédé selon la revendication 4, **caractérisé en ce que** l'hydrocarbure aromatique est choisi parmi le toluène, les o,m,p-xylènes, les o,m,p-hexafluoroxylènes et le 1,3-bis (trifluorométhyl) benzène.

7. Procédé selon la revendication 4, **caractérisé en ce que** le solvant de réaction est un mélange de THF et d'un hydrocarbure aromatique.

8. Procédé selon la revendication 7, **caractérisé en ce que** le solvant de réaction est un mélange de THF et d'un hydrocarbure aromatique choisi parmi le toluène et le 1,3-bis(trifluorométhyl)benzène.

9. Procédé selon la revendication 7 ou la revendication 8, **caractérisé en ce que** ledit mélange comprend de 20 à 60 % en poids de THF.

10. Procédé selon l'une quelconque des revendications précédentes, **caractérisé en ce que** l'halogénure de 3,5-bis(trifluorométhyl)phénylmagnésium est choisi parmi le bromure de 3,5-bis (trifluorométhyl) phénylmagnésium et le chlorure de 3,5-bis(trifluorométhyl)phénylmagnésium.

11. Procédé selon l'une quelconque des revendications précédentes, **caractérisé en ce que** le paraformaldéhyde solide est utilisé en quantité approximativement équimolaire ou en léger excès par rapport à l'halogénure de 3,5-bis (trifluorométhyl) phénylmagnésium.

12. Procédé selon la revendication 11, **caractérisé en ce que** l'excès molaire du paraformaldéhyde est inférieur ou égal à 5 % par rapport à l'halogénure de 3,5-bis(trifluorométhyl)phénylmagnésium.

13. Procédé selon l'une quelconque des revendications précédentes, **caractérisé en ce que** la température de réaction est comprise entre 30 et 90 °C.

14. Procédé selon l'une quelconque des revendications précédentes, **caractérisé en ce que** à la fin de la réaction l'adduit est hydrolysé par une solution aqueuse d'un acide minéral.

15. Procédé selon la revendication 14, **caractérisé en ce que** ledit acide minéral est choisi parmi l'acide chlorhydrique et l'acide sulfurique.

16. Procédé selon l'une quelconque des revendications précédentes, **caractérisé en ce que** l'alcool 3,5-bis(trifluorométhyl)benzyle est isolé par distillation ou cristallisation.

17. Procédé selon l'une quelconque des revendications précédentes, **caractérisé en ce que** l'alcool 3, 5-bis(trifluorométhyl)benzyle obtenu est utilisé comme un réactif afin d'obtenir un halogénure de 3,5-bis(trifluorométhyl)benzyle.

18. Procédé selon l'une quelconque des revendications précédentes, **caractérisé en ce que** ledit halogénure de 3,5-bis(trifluorométhyl)phénylmagnésium est obtenu en partant du 3,5-bis(trifluorométhyl)-1-halobenzène correspondant par traitement avec du magnésium dans un solvant choisi parmi les solvants cités dans les revendications 2 à 9.

19. Procédé selon les revendications 17 et 18, **caractérisé en ce que** :
(a) un halogénure de 3,5-bis(trifluorométhyl) phénylmagnésium est formé à partir d'un 3,5-bis (trifluorométhyl)halobenzène dans un solvant choisi parmi les éthers aliphatiques et un mélange d'éthers aliphatiques et d'hydrocarbures aromatiques ;
(b) du paraformaldéhyde solide est ajouté au mélange réactionnel ainsi obtenu ;
(c) l'alcool 3,5-bis(trifluorométhyl)benzyle ainsi obtenu est soumis à une réaction d'halogénation par HX où X est un halogénure, de manière facultative en présence d'acide sulfurique ;
(d) l'halogénure de 3,5-bis(trifluorométhyl) benzyle ainsi obtenu est isolé.
